**Europäisches Patentamt**

**European Patent Office**

**Office européen des brevets**

(19)

(11) Veröffentlichungsnummer: **0 101 561**
**B1**

(12) **EUROPÄISCHE PATENTSCHRIFT**

(45) Veröffentlichungstag der Patentschrift:
**04.01.89**

(51) Int. Cl.⁴: **C 07 D 239/30**

(21) Anmeldenummer: **83107011.5**

(22) Anmeldetag: **18.07.83**

(54) Verfahren zur Herstellung von 2,4,5,6-Tetrachlorpyrimidin.

(30) Priorität: **31.07.82 DE 3228712**

(43) Veröffentlichungstag der Anmeldung:
**29.02.84 Patentblatt 84/9**

(45) Bekanntmachung des Hinweises auf die Patenterteilung:
**04.01.89 Patentblatt 89/1**

(84) Benannte Vertragsstaaten:
**CH DE FR GB LI**

(56) Entgegenhaltungen:
**DE-A-1 933 784**
**US-A-3 075 980**

(73) Patentinhaber: **BAYER AG, Konzernverwaltung RP Patentabteilung, D-5090 Leverkusen 1 Bayerwerk (DE)**

(72) Erfinder: **Steffan, Guido, Dr., Herzogenfeld 52, D-5068 Odenthal (DE)**
Erfinder: **Zahl, Gero, Dr., Andreas- Gryphius- Strasse 7, D-5000 Köln 80 (DE)**

EP 0 101 561 B1

## Beschreibung

Die vorliegende Erfindung betrifft ein neues Verfahren zur Herstellung von 2, 4, 5, 6-Tetrachlorpyrimidin (TCP).

In der DE-PS-1 933 784 ist ein Verfahren zur Herstellung von Tetrachlorpyrimidin durch Umsetzung von Barbitursäure mit Phosphortrichlorid und Chlor in Phosporoxychlorid beschrieben.

Hierbei wird zunächst Barbitursäure in $POCl_3$ mit Chlor zu 5-Monochlorbarbitursäure umgesetzt.

Danach wird die gesamte, für die Herstellung von TCP benötigte $PCl_3$-Menge zugesetzt und nun entweder bei tiefer Temperatur Chlor zur Erzeugung des Chlorierungsagens $PCl_5$ eingeleitet, das danach durch Temperatursteigerung zur Reaktion mit der monochlorierten Barbitursäure gebracht wird, oder das Chlor wird bei nach einem bestimmten Programm gesteigerten Temperaturen allmählich eindosiert.

Die beiden vorgenannten Verfahrensvarianten sind wegen der erforderlichen Wärmeabfuhr zeitlich aufwendig, und die Ausbeuten schwanken in einem bestimmten Bereich.

Steigert man - um besser die Raktionswärme abführen zu können - die Raktionstemperatur zu früh, d.h. bevor bestimmte Chlormengen eingeleitet sind, so fallen die Ausbeuten deutlich ab.

Wegen der zeitweisen Viskosität der Ansätze durch die große Menge enthaltenen Phosphorpentachlorids läßt sich auch die Barbitursäure-Konzentration nicht mehr erhöhen.

Diese vorgenannten Fakten führen einer mäßigen Raum-Zeit-Ausbeute des TCP-Herstellungsverfahrens gemäß DE-PS-1 933 784.

Gegenstand der vorliegenden Erfindung ist nun ein Verfahren zur Herstellung von 2,4,5,6-Tetrachlorpyrimidin, dadurch gekennzeichnet, daß man in eine 5,5-Dichlorbarbitursäure und Phosphoroxychlorid enthaltende Mischung Phosphortrichlorid und Chlor in Gegenwart geringer Mengen eines tertiären Amins simultan bei Siedetemperatur der Mischung eindosiert und das entstehende Tetrachlorpyrimidin nach erfolgter Umsetzung abtrennt, isnbesondere destillativ.

Vorzugsweise arbeitet man dabei so, daß man gleichmäßig und gleichzeitig Phosphortrichlorid und Chlor während ca. 3 bis ca. 5 Stunden eindosiert und nach Beendigung der Zugabe noch bis zur Beendigung der HCl-Entwicklung unter Rückfluß erhitzt.

Bevorzugte tertiäre Amine sind Trialkylamine, insbesondere Triethylamin, die in Mengen von 0,01 - 0,1, bevorzugt 0,02 - 0,05 kg pro kg Barbitursäure bzw. 5,5-Dichlorbarbitursäure eingesetzt werden.

Vorzugsweise werden pro Mol eingesetzter 5,5-Dichlorbarbitursäure ca. 2,5 - 5 Mol und insbesondere ca. 3 Mol Phosphortrichlorid und ca. 1,5 bis ca. 3 Mol und insbesondere ca. 2 Mol Chlor eindosiert.

Diese Eindosierung kann überraschenderweise ohne Ausbeuteverluste bei Sidetemperatur der Mischung erfolgen, so daß die Raktionswärme durch das Sieden in recht kurzer Zeit z. B. durch Wahl eines entsprechend groß dimensionierten Kondensators abzuführen ist.

Dies führt zu erheblich verkürzten Reaktionszeiten. Da bei dem Gesamtherstellungsverfahren außerdem die Barbitursäure in größerer Konzentration eingesetzt werden kann, weil sich jeweils nur wenig die Viskosität steigerndes Phosphorpentachlorid in Ansatz befindet, wird auch die Raum-Ausbeute gesteigert, so daß sich die Raum-Zeit-Ausbeute gegenüber der des in DE-PS-1 933 784 beschriebenen Verfahrens mehr als verdoppeln läßt.

Hinzukommt, daß die Ausbeute an Tetrachlorpyrimidin wegen der klaren und gleichbleibenden Reaktionsführung stets bei 96 - 97 % d.Th., bezogen auf die Ausgangskomponente Barbitursäure, liegt.

Entsprechend gering ist damit auch der nach Destillation des Tetrachlorpyrimidins verbleibende Rückstand.

Die erfindungsgemäß mit $PCl_3$ und $Cl_2$ umzusetzende und 5,5-Dichlorbarbitursäure und Phosphoroxychlorid enthaltende Mischung kann neben den beiden vorgenannten Komponenten gegebenenfalls noch weitere z. B. 5-Monochlorbarbitursäure enthalten.

Die für die Durchführung des erfindungsgemäßen Verfahrens benötigte Mischung aus 5,5-Dichlorbarbitursäure und Phosphoroxychlorid wird vorzugsweise dadurch erhalten, daß man pro kg Barbitursäure 1 bis 10 kg und insbesondere 1,5 bis 3 kg Phosphoroxychlorid einsetzt und in die Mischung bei 30 bis 60°C und insbesondere 40 bis 50°C unter schwacher Kühlung und gutem Rühren zunächst pro kg Barbitursäure 0,3 bis 1,5 und insbesondere 0,5 bis 1,0 kg Chlor während 2 bis 5 und insbesondere 3 bis 4 Stunden so einleitet, daß im HCl-Abgas praktisch kein Chlor entweicht. Anschließend werden ohne Kühlung pro kg Barbitursäure 0,1 bis 0,5 und insbesondere 0,2 bis 0,4 kg Chlor während 1 bis 3 und insbesondere 1,5 bis 2 Stunden eingeleitet, wobei die Temperatur allmählich auf 60 bis 85 und insbesondere 75 bis 80°C ansteigt.

Tetrachlorpyrimidin ist bekannt als wichtiges Zwischenprodukt zur Herstellung von Raktivfarbstoffen (siehe z. B. GB-PS 902 618) und als Ausgangsprodukt zur Herstellung einer weiteren wichtigen Reaktivkomponente Trifluor-monochlorpyrimidin (siehe z. B. FR-PS-1 546 305). Darüber hinaus findet TCP Verwendung als fungicides und sporicides Mittel (US-PS-3 227 612).

Bei der Herstellung der Reaktivfarbstoffe wird dabei ein chromophores System mit TCP unter Abspaltung von HCl und unter Einführung des Trichlorpyrimidinyl-Restes zum Erhalt von Reaktivfarbstoffen umgesetzt.

Das im folgenden beschrieben Herstellungsbeispiel soll das erfindungsgemäße Verfahren charakterisieren.

**Herstellungsbeispiel:**

In eine Mischung von
505 kg Phosphoroxychlorid und
219 kg Barbitursäure werden unter gutem Rühren (80 - 100 Umin) und schwacher Kühlung bei von 40 auf 50°C steigender Temperatur.
170 kg Chlor in 3,5 - 4 Stunden so eingeleitet, daß im HCl-Abgas praktisch kein Chlor entweicht. Danach werden ohne Kühlung weitere
70 kg Chlor in 1,5 - 2 Stunden eingeleitet, wobei man die Temperatur allmählich auf 75 - 80°C steigen läßt.

Der so hergestellten Lösung / Suspension von 5,5-Dichlorbarbitursäure in $POCl_3$ werden
30 kg Posphortrichlorid und
5,7 kg Triethylamin zugesetzt.
Dann werden in ca. 4 Stunden gleichzeitig und gleichmäßig über die Zeit verteilt
694 kg Phosphortrichlorid und
250 kg Chlor in den Ansatz eindosiert, wobei die erhebliche Reaktionswärme durch Sieden unter Rückfluß abgeführt wird (Ansatztemperatur 100 - 107°C).

Nach Beedigung des Chloreinleitens wird noch für 1,5 - 2 Stunden bis zur Beendigung der HCl-Entwicklung zum Sieden unter Rückfluß erhitzt.
Durch die nachfolgende destillative Aufarbeitung werden.
1.286 kg Phosphoroxychlorid und
362 kg Tetrachlorpyrimidin mit einem Gehalt von 99,3 % erhalten, was einer Ausbeute von 96,4 % d.Th. entspricht.

Es verbleibt ein Destillationsrückstand von 17,7 kg. Die Konstanz der Ausbeute wurde in zahlreichen Ansätzen bestätigt.
Die gesamte Reaktionszeit (ohne Destillation) beträgt 11 - 12 Stunden bei einem Reaktionsvolumen von ca. 1.000 l. Die Raum-Zeit-Ausbeute beträgt also etwa 30 kg Tetrachlorpyrimidin pro $m^3$ und Stunde.
Pro kg Tetrachlorpyrimidin sind bei der destillativen Aufarbeitung ca. 3,6 kg $POCl_3$ zu destillieren.

**Vergleichsbeispiel nach DE-PS-1 933 784 (analog Beispiel 1)**

In eine Mischung von
707 kg Phosphoroxychlorid
4 kg Phosphortrichlorid und
141 kg Barbitursäure werden bei etwa 50 - 55°C
94 kg Chlor in ca. 2,5 Stunden so eingeleitet, daß im HCl-Abgas praktisch kein Chlor enthalten ist.

Dann werden bei 20°C
463 kg Phosphortrichlorid und
2,2 kg Triethylamin zugesetzt und
243 kg Chlor unter Kühlung bei 20 - 30°C aufgeleitet, was ca. 9 Stunden erfordert.

Danach wird die Mischung in ca. 5 Stunden unter starker HCl-Entwicklung allmählich bis zur Rückflußtemperatur (110 - 115°C) erwärmt.

Danach wird der Ansatz noch 1 Stunde auf Rückflußtemperatur gehalten, bis die HCl-Entwicklung völlig beendet ist.
Durch die nachfolgende destillative Aufarbetung werden
1.204 kg Phosphoroxychlorid und
230 kg Tetrachlorpyrimidin mit einem Gehalt von 99,2 % erhalten, was einer Ausbeute von 95 % d.Th. entspricht. Es verbleibt ein Destillationsrückstand von 25,5 kg.

Die gesamte Reaktionszeit (ohne Destillation) beträgt 17,5 - 18 Stunden bei einem Reaktionsvolumen von ca. 1.000 l.
Die Raum-Zeit-Ausbeute beträgt also etwa 13 kg Tetrachlorpyrimidin pro $m^3$ und Stunde.
Pro kg Tetrachlorpyrimidin sind bei der destillativen Aufarbeitung ca. 5,3 kg $POCl_3$ zu destillieren.

**Patentansprüche**

1. Verfahren zur Herstellung von 2, 4, 5, 6-Tetrachlorpyrimidin, dadurch gekennzeichnet, daß man in eine 5,5-Dichlorbarbitursäure und Phosphoroxychlorid enthaltende Mischung Phosphortrichlorid und Chlor in Gegenwart geringer Mengen eines tertiären Amins simultan bei Siedetemperatur der Mischung eindosiert und das entstehende Tetrachlorpyrimidin nach erfolgter Umsetzung abtrennt.

2. Verfahren nach Anspruch 1, dadurch gekennzeichnet, daß man die Umsetzung bei 90 bis 107° C durchführt.

3. Verfahren nach Anspruch 1, dadurch gekennzeichnet, daß man gleichmäßig und gleichzeitig Phosphortrichlorid und Chlor während ca. 3 bis ca. 5 Stunden eindosiert und nach Beendigung der Zugabe noch bis zur Beendigung der HCl-Entwicklung unter Rückfluß erhitzt.

4. Verfahren nach Anspruch 1, dadurch gekennzeichnet, daß man als tertiäres Amin Trialkylamin einsetzt.

5. Verfahren nach Anspruch 1, dadurch gekennzeichnet, daß man als tertiäres Amin Triethylamin verwendet.

6. Verfahren nach Anspruch 1, dadurch gekennzeichnet, daß pro Mol eingesetzter 5,5-Dichlorbarbitursäure ca. 2,5 bis 5 Mol und insbesondere ca. 3 Mol Phosphortrichlorid und ca. 1,5 bis ca. 3 Mol und insbesondere ca. 2 Mol Chlor eindosiert werden.

7. Verfahren nach Anspruch 1, dadurch gekennzeichnet, daß man aus dem Reaktionsgemisch das entstehende 2, 4, 5, 6-Tetrachlorpyrimidin destillativ abtrennt.

**Claims**

1. Process for the preparation of 2, 4, 5, 6-tetrachloropyrimidine, characterized in that phosphorus trichloride and chlorine are metered simultaneously, in the presence of small amounts of a tertiary amine and at the boiling point of the mixture, into a mixture containing 5,5-dichlorobarbituric acid and phosphorus oxychloride, and the resulting tetrachloropyrimidine is separated off when the reaction is complete.

2. Process according to Claim 1, characterized in that the reaction is carried out at 90 to 107° C.

3. Process according to Claim 1, characterized in that phosphorus trichloride and chlorine are metered in uniformly and simultaneously for approximately 3 to approximately 5 hours and, after the addition is complete, the mixture is heated further under reflux until the evolution of HCl is complete.

4. Process according to Claim 1, characterized in that the tertiary amine employed is a trialkylamine.

5. Process according to Claim 1, characterized in that the tertiary amine used is triethylamine.

6. Process according to Claim 1, characterized in that approximately 2.5 to 5 moles, especially approximately 3 moles, of phosphorus trichloride and approximately 1.5 to approximately 3 moles, especially approximately 2 moles, of chlorine are metered in per mole of 5,5-di-chlorobarbituric acid employed.

7. Process according to Claim 1, characterized in that the 2, 4, 5, 6-tetrachloropyrimidine which is formed is removed from the reaction mixture by distillation.

**Revendications**

1. Procédé de production de 2, 4, 5, 6-tétrachloropyrimidine, caractérisé en ce qu'on introduit simultanément du trichlorure de phosphore et du chlore en présence de faibles quantités d'une amine tertiaire dans un mélange contenant de l'acide 5,5-dichlorobarbiturique et de l'oxychlorure de phosphore, à la température d'ébullition du mélange et une fois la réaction terminée, on sépare la tétrachloropyrimidine produite.

2. Procédé suivant la revendication 1, caractérisé en ce qu'on conduit, la réaction à une température de 90 à 107°C.

3. Procédé suivant la revendication 1, caractérisé en ce qu'on introduit uniformément et simultanément du trichlorure de phosphore et du chlore pendant environ 3 à environ 5 heures et une fois l'addition terminée, on chauffe encore au reflux jusqu'à ce que le dégagement de HCl soit terminé.

4. Procédé suivant la revendication 1, caractérisé en ce qu'on utilise comme amine tertiaire une trialkylamine.

5. Procédé suivant la revendication 1, caractérisé en ce qu'on utilise comme amine tertiaire la triéthylamine.

6. Procédé suivant la revendication 1, caractérisé en ce qu'on introduit, par mole d'acide 5,5-dichlorobarbiturique utilisé, environ 2,5 à 5 moles et notamment environ 3 moles de trichlorure de phosphore, et environ 1,5 à environ 3 moles et notamment environ 2 moles de chlore.

7. Procédé suivant la revendication 1, caractérisé en ce que la 2, 4, 5, 6-tétrachloropyrimidine produite est séparée par distillation du mélange réactionnel.